# EUROPÄISCHE PATENTSCHRIFT

(11) **EP 0 201 905 B1**
(45) Veröffentlichungstag und Bekanntmachung des Hinweises auf die Patenterteilung: **27.01.1993**
(21) Anmeldenummer: 86106441.8
(22) Anmeldetag: 12.05.1986
(51) Int. Cl.: A61B 17/58

(54) **Dauerspannbares Kunststoffband mit Selbsthalterung**
Self-retaining permanently tensionable synthetic tie
Bande synthétique supportant une contrainte permanente avec dispositif autoblocant

(30) Priorität: 13.05.1985 DE 3517204
(43) Veröffentlichungstag der Anmeldung: 20.11.1986
(73) Patentinhaber: Hauer, Gerald Dr., D-82362 Weilheim (DE)
(72) Erfinder: Hauer, Gerald Dr., D-82362 Weilheim (DE)
(74) Vertreter: Patentanwälte Dr. Solf & Zapf

(56) Entgegenhaltungen:
- DE-B- 1 089 116
- DE-U- 1 916 924
- FR-A- 2 357 229
- US-A- 2 760 488

## Beschreibung

Die Erfindung betrifft ein dauerspannbares Kunststoffband mit Selbsthalterung zur Verwendung in Chirurgie und Orthopädie. der im Oberbegriff des Anspruchs 1 bzw. 6 genannten Art.

Es ist seit langem bekannt, für Operationen im unfallchirurgischen und orthopädischen Bereich Metalldrähte für Zuggurtungen und Cerclagen zu verwenden.

Bei der Cerclage umschließt ein Draht reponierte Knochenfragmente und hält sie temporär oder dauernd zusammen. Die Zuggurtung soll muskuläre Zugspannungen und Belastungskräfte neutralisieren.

Der seit über hundert Jahren dafür gebräuchliche Bindedraht beweist insbesondere bei der konventionellen Zuggurtung zusätzlich zu den biomechanischen Mängeln Nachteile auf, die auf seinen mechanischen Eigenschaften beruhen. Er bewirkt keine funktionelle Stabilität; Festigkeit des Knochen-Metallverbundes durch Kompresson wäre mit Draht nur erreichbar, wenn dieser dauerhaft vorgespannt werden könnte. Das ist nicht erreichbar, da der Bindedraht relativ steif und unelastisch ist und sich bereits bei Belastungen von 200 N/mm 0̸ 1 mm streckt. Zudem können die Drahtenden nur schlecht miteinander verbunden werden. Durch Verdrehen der Enden läßt sich Bindedraht nicht spannen, weil er sich im Zwirbel dehnt und keine Zugkraft weitergeben kann. Bricht der Draht im Zwirbel, muß neu reponiert werden. Bindedraht ist daher für Cerclagen und wirksame Zuggurtungen nur wenig geeignet.

Zur Überwindung dieser Nachteile wurde in neuerer Zeit die Seilosteosynthese eingeführt, die zum ersten Mal praktisch brauchbare Zuggurtungen bei Operationen zugänglich machte. Nach dieser von Labitzke entwickelten Methode werden zur Aufbringung der Zugkraft Drahtseile verwendet, die sich kontrollierbar spannen und befestigen lassen. Die Drahtseile werden mit einer plombenähnlichen Preßklemme unter Spannung gehalten. Jedoch ergibt sich bei Cerclagen mit Drahtseilen das Problem, daß durch die notwendige Materialdicke der zur Anpressung der Preßklemme an die Drahtseile notwendigen Zange die Preßklemme beim Preßvorgang nicht am Knochen anliegt. Nach Entfernen der Zange fällt die Preßklemme auf die Kortikalis herab. So entsteht ein Abstand der Cerclage vom Knochenumfang, so daß die Cerclage zu weit wird. Entsprechend läßt die interfragmentäre Kompression nach oder geht sogar ganz verloren. Zur Vermeidung dieses Nachteils ist es notwendig, spezielle Seilbrücken zwischen dem Cerclageseil und der Knochenoberfläche einzusetzen.

Die Seilosteosynthese bietet zwar gegenüber der herkömmlichen Cerclage- und Zuggurtungs-Osteosynthese mit Bindedraht bereits Verbesserungen, erfordert jedoch zur Verankerung der Drahtseile am Knochen das Einsetzen von über dessen Außenumfang vorstehenden Schrauben. Auch die Seilschuhe und Preßklemmen sowie die nach Verspannen und Abschneiden der Drahtseile stehenbleibenden Seilenden stehen über die Knochenaußenfläche vor, was zu Komplikationen führen kann. Auch läßt die Biokompatibilität der verwendeten Metallwerkstoffe zu wünschen übrig. Außerdem ist die Seilosteosynthese hinsichtlich der verwendeten Materialien wie auch ihrer operativen Verwendung aufwendig.

Ein dauerspannbares Kunststoffband der eingangs genannten Art ist aus der FR-A-2 357 229 bekannt. Der Spannippel dieses bekannten Bandes ist als im wesentlichen rechtwinkliges Vielflach ausgebildet, wobei das Band an eine Seite dieses Vielflachs angebunden ist. Der den Spannippel durchsetzende Bandkanal verläuft in etwa lotrecht zur Bandlängsrichtung. Beim bestimmungsgemäßen Einsatz steht der Spannippel dieses bekannten dauerspannbaren Kunststoffbandes unvermeidlich von der Knochenaußenfläche vor und ist damit Anlaß für postoperative Komplikationen.

Aus der DE-A-1 089 116 ist eine Knochenschraube zur Verbindung der Enden von zwei Knochen, wie Schienen- und Wadenbein, bekannt, die ein gabelähnliches Pfannenlager zur kugelgelenkartigen Abstützung auf einem Knöchel bilden. Die Knochenschraube weist einen Flachkopf auf sowie ein Mutterstück, wobei die Schraube einen starr und fest damit verbundenen schildförmigen Kopf aufweist, dessen Tangentialebene im Berührungspunkt mit dem Schraubenschaft einen spitzen Winkel mit der Schraubenachse bildet. Das Mutterstück weist die Form eines runden gewölbten Tellers mit von einem Mittelloch ausgehenden radialen Einschnitten und dadurch gebildeten federnden Zungen auf.

Aufgabe der Erfindung ist es daher, ein Kunststoffband der eingangs genannten Art zu schaffen, das unaufwendig herstellbar und operativ anwendbar ist und ohne nachfolgende Komplikationen eine zuverlässige bleibende Zugspannung auf die in Position zu fixierenden Teile auszuüben gestattet.

Zur Lösung dieser Aufgabe ist das dauerspannbare Kunststoffband der eingangs genannten Art erfindungsgemäß mit den im kennzeichnenden Teil des Anspruchs 1 bzw. mit den im Kennzeichnurden Teil des Anspruchs 6 definierten Merkmalen ausgestattet

Der das erfindungsgemäße Kunststoffband im angespannten Zustand haltende Spannippel kann in den Knochen versenkt werden, so daß kein Überstehen mit den entsprechenden Funktionsstörungen auftreten kann. Das Kunststoffband eignet sich sowohl für Cerclagen wie auch für Zuggurtungen. Damit ist auch bei unzugänglichen Knochenabschnitten eine feste Verbindung möglich und eine groß angelegte Knochenfreilegung kann unterbleiben.

Vorteilhafte weitere Ausgestaltungen des Kunststoffbandes sind in den abhängigen Ansprüchen angegeben.

Nach der in den Ansprüchen 1 bis 5 definierten, bevorzugten Ausführungsform des Kunststoffbandes wird dieses durch eine Durchbohrung geführt, die die zu verbindenden Knochenfragmente durchsetzt. Die an einem Ende des Kunststoffbandes vorgesehene Verdickung tritt beim Anspannen des Kunststoffbandes teilweise in die Durchbohrung ein und bildet ein die Zugspannung auf das Knochenfragment übertragendes Gegenlager. Am anderen Ende der Durchbohrung tritt das das Kunststoffband unter Spannung haltende Spannmittel ebenfalls in die Durchbohrung ein. Nachdem die gewünschte Zugspannung erreicht ist, können die überstehenden Teile von Verdickung und Spannippel mit der Knochenaußenfläche fluchtend abgeschnitten werden, so daß sich trotz zugfester Verbindung der Knochenfragmente kein störender Überstand ergibt. Bei der in den Ansprüchen 6 und 7 beschriebenen bevorzugten Ausführungsform umgreift das Kunststoffband den Knochenaußenumfang; der Spannippel kann leicht in den Knochen versenkt werden und tritt durch seine abgeflachte Außenfläche kaum über dessen Außenumfang vor.

Das erfindungsgemäße dauerspannbare Kunststoffband kann in der Chirurgie des Haltungs- und Bewegungsapparates z.B. in folgenden Fällen verwendet werden:
Provisorische Fixierung von Frankturen (z.B. bei der offenen Marknagelung);
Osteosynthesen (Goetze-Cerclagen, Zuggurtungen, Cerclagen, Hemicerclagen; die Indikation hierzu kann ausgedehnt werden, da eine feste Verankerung möglich ist);
Veränderung der distalen Kortikalisausbrüche bei der Federnagelung nach Ender und Simon-Weidner;
Unterstützung von Bandplastiken (Schulter-Eck-Gelenk, Sprunggelenk, Knie-Kreuzband);
Adaptation und Wiedervereinigung nach Osteotomien(z.B. Sternum, insbesondere auch bei Herzeingriffen, Trochanter major);
Erleichterung des Verschlusses der Thoraxwand durch stabile Adaptation benachbarter Rippen;
Verbesserung der Rippenosteosynthese, indem die üblichen Judet-Platten aus Kunststoff nachgebildet werden und die sonst zur Verankerung umgebogenen Metallamellen durch die erfindungsgemäßen Kunststoffbänder ersetzt werden.

Im folgenden werden bevorzugte Ausführungsformen der Erfindung an Hand der beigefügten Zeichnungen weiter erläutert.

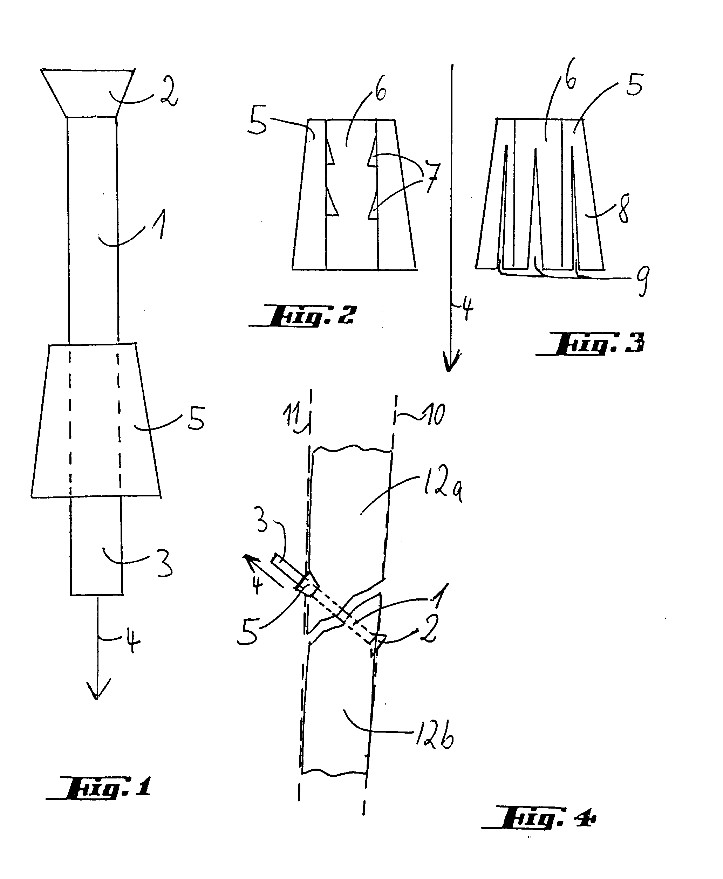

Es zeigen: Figur 1 eine schematische Ansicht des erfindungsgemäßen Kunststoffbandes;
Figur 2 einen längsgeschnittenen Spannippel;
Figur 3 einen längsgeschnittenen abgewandelten Spannippel;
Figur 4 schematisch die Anwendung des Kunststoffbandes bei Zuggurtung eines Knochenbruchs;
Figur 5 ein abgewandeltes Kunststoffband gemäß der Erfindung und
Figur 6 einen längsgeschnittenen dritten Spannippel.

Das in Figur 1 in einer bevorzugten Ausführungsform gezeigte Kunststoffband 1 weist glatte Außenflächen auf; dies gestattet die stufenlose Anspannung des Kunststoffbandes 1 und damit die exakte Einstellung der gewünschten Zugspannung. Das Kunststoffband 1 trägt an einem Ende eine Verdickung 2 , die unter Zugspannung des Kunststoffbandes 1 ein Gegenlager bildet. Das Kunststoffband 1 ist durch einen Spannippel 5 geführt, der zylindrisch ausgebildet ist oder sich konisch gegen die Spannrichtung 4 des Kunststoffbandes 1 verjüngt. Das Kunststoffband 1 ist an dem longitudinalen Endabschnitt geringeren Durchmessers des Spannippels 5 in diesen eingeführt und tritt an seinem longitudinalen Endabschnitt größerem Durchmessers wieder aus dem Spannippel 5 aus, wobei es einen Überstand 3 bildet. Dieser dient zum Spannen des Kunststoffbandes 1, z.B. zum Ansetzen geeigneter Spannwerkzeuge, und wird nach Erreichen der gewünschten Zugspannung abgeschnitten. Je nach Einsatzzweck des Kunststoffbandes 1 ist die an seinem Ende vorgesehene Verdickung 2 entweder so ausgestaltet, daß sie sich in Spannrichtung des Kunststoffbandes 1 konisch verjüngt oder zylindrisch ausgebildet ist oder ist als quer zur Längsrichtung des Kunststoffbandes 1 mit diesem fest verbundene flache Scheibe ausgebildet.

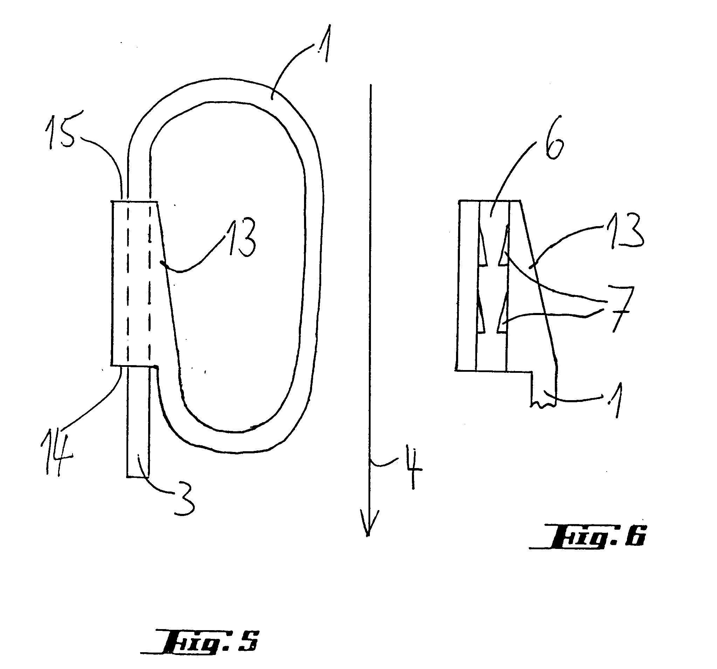

Wie Figur 2 zeigt, weist der Spannippel 5 einen ihn in Längsrichtung durchsetzenden Bandkanal 6 auf, durch den das Kunststoffband 1 geführt werden kann. Dabei füllt es den Bandkanal 6 im wesentlichen aus, so daß ein störungsfreies Zusammenwirken von Kunststoffband 1 und Spannnippel 5 gewährleistet ist. Auf seiner Innenfläche ist der Spannippel 5 mit mehreren, in den Bandkanal 6 vortretenden Gesperrnasen oder -rippen 7 versehen, die in Spannrichtung 4 des Kunststoffbandes gesehen mit relativ geringer Steigerung in den Bandkanal 6 vortreten, gegen die Spannrichtung 4 gesehen aber im wesentlichen quer zur Spannrichtung 4 in den Bandkanal 6 vortretende Stufenflächen bilden. Da das Kunststoffband 1 den Bandkanal 6 im wesentlichen füllt, werden die Gesperrnasen 7 beim Einführen des Kunststoffbandes 1 aus dem Bandkanal 6 zurückgedrängt, indem das Kunststoffband (1) an ihnen entlanggleitet. Die Gesperrnasen oder -rippen 7 pressen sich dabei in das Kunststoffband 1 ein. Das Kunststoffband 1 wird an einer Bewegung gegen die Spannrichtung 4 durch die Gesperrnasen 7 gehindert, die sich bei einer solchen Bewegung mit ihren Stufenflächen und deren Schärfen, in den Bandkanal 6 hervorragenden Kanten in das Kunststoffband 1 eingraben.

Ein abgewandelter Spannippel 5 ist in Figur 3 im Längsschnitt gezeigt. Er ist durch mehrere, von seinem verdickten Endabschnitt ausgehende Schlitre 9 auf einem Teil seiner Längserstreckung in Nippelsegmente 8 unterteilt. Beim Einsetzen des Spannippels 5 in eine Durchbohrung, wie unten an Hand der Figur 4 näher erläutert, wird der Spannippel 5 unter der Zugspannung des Kunststoffbandes 1 vom Umfang her zusammengedrückt. Dabei gestatten die Schlitze 9, daß die Nippelsegmente 8 unter Verengung des Bandkanals 6 einwärts geschoben werden und dabei, ähnlich wie die Gesperrnasen 7 des oben beschriebenen Spannippels 5, das Kunststoffband 1 festklemmen.

Zur Zuggurtung von Knochenfrakturen wird, wie Figur 4 schematisch zeigt, zunächst eine in etwa geradlinige Durchbohrung der unter Zugspannung zu verbindenden Knochenfragmente 12 a und 12 b vorgenommen. Nachfolgend wird das Kunststoffband 1 durch die Durchbohrung der Fragmente geführt, bis seine Verdickung 2 am Außenumfang des einen Knochenfragments 12 b in die Durchbohrung eintritt. Der Spannippel 5 wird auf das Überstandsende 3 des Kunststoffbandes 1 aufgeschoben, welches am Außenumfang des anderen Knochenfragments 12 a aus der Durchbohrung vortritt` Dabei weist sein konisch verjüngter longitudinaler Endabschnitt mit geringerem Durchmesser in Richtung auf die Verdickung 2.

Der Spannippel 5 wird nun auf dem Kunststoffband 1 soweit in Richtung auf die Fertigung 2 verschoben, daß sein Endabschnitt geringeren Durchmessers am Außenumfang des Knochenfragments 12 a ebenfalls in die Durchbohrung eintritt. Nachfolgend wird das Kunststoffband 1 gegenüber dem Spannippel 5 solange in Spannrichtung 4 angezogen, bis die für die Zuggurtung gewünschte Zugspannung erreicht ist. In dieser Stellung wird es entweder durch die Gesperrnasen 7 oder durch die Nippelsegmente 8 festgeklemmt. Die über die Außenumfangsflächen 10,11 der Knochenfragmente 12 a bzw. 12 b vortretenden Teile des Überstands 3 des Kunststoffbands 1 und des Spannippels 5 einerseits, der Verdickung 2 andererseits können jetzt so abgeschnitten werden, daß sie mit den Außenumfangsflächen der Knochenfragmente 12 a bzw. 12 b fluchten. Es versteht sich, daß der Spannippel 5 so mit Gesperrnasen 7 bzw. Schlitzen 9 und dadurch gebildeten Nippelsegmenten 8 versehen sein muß, daß ein sicheres Einklemmen des Kunststoffbandes 1 durch den Bereich des Spannippels 5 gewährleistet ist, der sich innerhalb der Durchbohrung des Knochenfragments 12 befindet.

Im folgenden wird eine zweite bevorzugte Ausführungsform des Kunststoffbandes 1 näher erläutert, die für umgreifende Cerclagen ausgelegt ist. Bei dieser in Figur 5 gezeigten bevorzugten Ausführungsform ist das Kunststoffband 1 an einem Ende fest mit dem in Spannrichtung hinteren Endabschnitt 14 des Spannippels 13 verbunden. Der Spannnippel 13 muß bei dieser Ausführungsform nicht konisch sein, sondern kann vielmehr einen über seine Längserstreckung gleichbleibenden Durchmesser aufweisen. Er ist bevorzugt so ausgebildet, daß eine glatte Anlagefläche an den zu halternden Knochen gebildet wird. Er ist z.B. auch abgeflacht ausgebildet.

Wie Figur 6 zeigt, wird der Spannippel 13 ebenfalls von einem Bandkanal 6 in Längsrichtung durchsetzt; das dauerelastische Kunststoffband 1 wird zur Anlegung der Cerclage um die zu verbindenden Teile (z.B. Knochenfragmente) geführt und dann mit seinem freien Ende in den in Spannrichtung 4 vorderen Endabschnitt 15 des Spannippels 13 eingeführt. Dieser ist auf seiner Innenfläche mit wenigstens einer In den Bandkanal 6 vortretenden, das eingeführte Kunststoffband 1 an einer Bewegung gegen die Spannrichtung 4 hindernden Gesperrnase 7 versehen. Nach Anliegen des Kunststoffbandes 1 wird dieses, z.B. durch Ansetzen eines geeigneten Werkzeugs an den aus dem Endabschnitt 14 des Spannippels 13 austretenden Überstand 3, solange gegenüber dem Spannippel 13 angezogen, bis es sich unter Erreichen der gewünschten Zugspannung fest an den Außenumfang der zu verbindenden Teile angelegt hat.

Der Spannippel 13 wird in eine vorher geschaffene Vertiefung im Außenumfang eines der unter Zugspannung zu verbindenden Teile eingelassen und dadurch in diesem gegen ein Verschieben gesichert. Seine nach außen gewandte Fläche kann so abgeflacht und ggf. gekrümmt sein, daß der Spannippel 13 nur unwesentlich über den Außenumfang des Teils vorsteht.

Das Kunststoffband 1 wie auch der Spannippel 5,13 bestehen aus biokompatiblem Material. Es kann vorgesehen werden, diese Teile aus resorbierbaren Polymeren auszubilden, so daß sie nach Verheilen der Fraktur vom Körper resorbiert werden. Andererseits lassen sich das Kunststoffband 1 und der Spannnippel 5 auch aus dauerkompatiblem Material anfertigen, beispielsweise aus dem im Handel unter der Bezeichnung "Gortex" erhältlichen Material, das beim Heilungsprozeß von Knochensubstanz durchwachsen wird und auf Dauer im Körper verbleiben kann.

## Patentansprüche

1. Dauerspannbares Kunststoffband mit Selbsthalterung zur Verwendung in Chirurgie und Orthopädie, mit einem von einem Bandkanal (6) in Längsrichtung durchsetzten Spannippel (5,13), einem durch den Bandkanal (6), diesen im wesentlichen ausfüllend führbares dauerelastisches Kunststoffband (1) und das Kunststoffband (1), auch unter Zugspannung durch Verengung des Bandkanals (6) an einer Bewegung gegen die Spannrichtung hindernden Sperrvorrichtungen (7,8), wobei das Kunststoffband (1) und der Spannippel (5,13) aus biokompatiblem, resorbierbarem oder dauerkompatiblem Material bestehen,
dadurch **gekennzeichnet**,
daß das Kunststoffband (1) glatte Außenflächen aufweist, das Kunststoffband (1) an einem Ende eine Verdickung (2) zur Bildung eines Gegenlagers bei Zugspannung aufweist, der Spannippel (5) sich konisch gegen die Spannrichtung des Kunststoffbandes (1) verjüngt oder zylindrisch ausgebildet ist, und die Verdickung (2) in Spannrichtung des Kunststoffbandes (1) sich konisch verjüngt.

2. Band nach Anspruch 1,
dadurch **gekennzeichnet**,
daß die Verdickung (2) des Kunststoffbandes (1) als quer zur Längsrichtung des Kunststoffbandes (1), mit diesem fest verbundene flache Scheibe ausgebildet ist.

3. Band mach Anspruch 1 oder 2,
dadurch **gekennzeichnet**,
daß der Spannippel (5) auf seiner Innenfläche mit in wenigstens einer in den Bandkanal (6) vortretenden, das eingeführte Kunststoffband (1) an einer Bewegung gegen die Spannrichtung hindernden Gesperrnase (7) versehen ist.

4. Band nach einem der Ansprüche 1 bis 3,
dadurch **gekennzeichnet**,
daß der Spannippel (5) wenigstens einen im wesentlichen in dessen Längsrichtung verlaufenden Schlitz (9) aufweist.

5. Band nach Anspruch 4,
dadurch **gekennzeichnet**,
daß der Spannippel (5) durch mehrere, von seinem verdickten Endabschnitt ausgehende Schlitze (9) auf einem Teil seiner Längserstreckung in Nippelsegmente (8) unterteilt ist.

6. Dauerspannbares Kunststoffband mit Selbsthalterung zur Verwendung in Chirurgie und Orthopädie, mit einem von einem Bandkanal (6) in Längsrichtung durchsetzten Spannippel (5,13), einem durch den Bandkanal (6), diesen im wesentlichen ausfüllend, führbares dauerelastisches Kunststoffband (1), und das Kunststoffband (1) auch unter Zugspannung durch Verengung des Bandkanals (6) an einer Bewegung gegen die Spannrichtung hindernden Sperrvorrictungen (7,8), wobei das Kunststoffband (1) und der Spannippel (5,13) aus biokompatiblem, resorbierbarem oder dauerkompatiblem Material bestehen und wobei das Kunststoffband (1) an einem Ende fest mit dem in Spannrichtung hinteren Endabschnitt (14) des Spannippels (13) verbunden ist,
dadurch **gekennzeichnet**,
daß das Kunststoffband (1) glatte Außenflächen aufweist, und der Spannippel (13) konisch gegen die Spannrichtung des Kunststoffbandes (1) verjüngt oder zylindrisch ausgebildet ist.

7. Band nach Anspruch 6,
dadurch **gekennzeichnet**,
daß der Spannippel (13) auf seiner Innenfläche mit wenigstens einer in den Bandkanal (6) vortretenden, das eingeführte Kunststoffband (1) an einer Bewegung gegen die Spannrichtung hindernde Gesperrnase (7) versehen ist und gegebenenfalls eine abgeflachte oder gekrümmte Außenseite aufweist.

## Claims

1. A self-retaining permanently tensionable synthetic tie for use in surgery and orthopaedics, having a tensioning nipple (5, 13) through which a tie passage (6) passes in the longitudinal direction thereof, a permanently elastic synthetic tie (1) guidable through, and substantially filling, the tie passage (6), and locking devices (7, 8) which prevent the synthetic tie (1) from moving in the opposite direction to the direction of tensioning even when under tensile stress by narrowing of the tie passage (6), wherein the synthetic tie (1) and the tensioning nipple (5, 13) are made from bio-compatible, reabsorbable or permanently compatible material, characterised in that the synthetic tie (1) has smooth outer surfaces, the synthetic tie (1) has a thickened portion (2) at one end for forming an abutment during tensile stress, the tensioning nipple (5) tapers conically in the opposite direction to the direction of tensioning of the synthetic tie (1) or is of cylindrical construction, and the thickened portion (2) tapers conically in the direction of tensioning of the synthetic tie (1).

2. A tie as claimed in claim 1, characterised in that the thickened portion (2) of the synthetic tie (1) is in the form of a flat disc rigidly connected to the synthetic tie (1) transversely to the longitudinal direction of the latter.

3. A tie as claimed in claim 1 or 2, characterised in that the interior surface of the tensioning nipple (5) is provided with at least one locking lug (7) which projects into the tie passage (6) and which prevents the inserted synthetic tie (1) from moving in the opposite direction to the direction of tensioning.

4. A tie as claimed in one of the claims 1 to 3, characterised in that the tensioning nipple (5) has at least one slot (9) extending in the longitudinal direction thereof.

5. A tie as claimed in claim 4, characterised in that the tensioning nipple (5) is subdivided over a portion of its longitudinal extent into nipple segments (8) by a plurality of slots (9) commencing from its thickened end portion.

6. A self-retaining permanently tensionable synthetic tie for use in surgery and orthopaedics, having a tensioning nipple (5, 13) through which a tie passage (6) passes in the longitudinal direction thereof, a permanently elastic synthetic tie (1) guidable through, and substantially filling, the tie passage (6), and locking devices (7, 8) which prevent the synthetic tie (1) from moving in the opposite direction to the direction of tensioning, even when under tensile stress, by narrowing of the tie passage (6), wherein the synthetic tie (1) and the tensioning nipple (5, 13) are made from bio-compatible, reabsorbable or permanently compatible material, and wherein one end of the synthetic tie (1) is rigidly connected to the rear end portion (14) of the tensioning nipple (13) in the direction of tensioning, characterised in that the synthetic tie (1) has smooth outer surfaces, and the tensioning nipple (13) tapers conically in the opposite direction to the direction of tensioning of the synthetic tie (1) or is of cylindrical construction.

7. A tie as claimed in claim 6, characterised in that the interior surface of the tensioning nipple (13) is provided with at least one locking lug (7) which projects into the tie passage (6) and which prevents the inserted synthetic tie (1) from moving in the opposite direction to the direction of tensioning, and said tensioning nipple possibly has a flattened or curved outer side.

## Revendications

1. Bande synthétique supportant une contrainte permanente, avec dispositif autoblocant, pour utilisation en chirurgie et en orthopédie, comportant un anneau de tension (5, 13) traversé, dans le sens longitudinal, par un canal de bande (6), une bande synthétique (1) à élasticité permanente, guidée dans le canal de bande (6) et remplissant essentiellement ce dernier, et des dispositifs de tension (7, 8) empêchant, par un rétrécissement du canal de bande (6), la bande synthétique (1) de se déplacer dans le sens opposé à la direction de la tension, même dans le cas d'un effort de traction, étant entendu que la bande synthétique (1) et l'anneau de tension (5, 13) sont constitués de matière biocompatible, résorbable ou compatible de façon permanente caractérisée en ce que la bande synthétique (1) présente des surfaces lisses, en ce que la bande synthétique (1) présente à une extrémité une surépaisseur (2) formant contre-appui en cas de traction, en ce que l'anneau de tension (5) se rétrécit en formant un cône dans le sens opposé à la direction de la tension de la bande synthétique (1) ou est réalisé cylindrique, et en ce que la surépaisseur (2) se rétrécit en forme de cône dans la direction de la tension de la bande synthétique (1).

2. Bande suivant la revendication 1, caractérisée en ce que la surépaisseur (2) de la bande synthétique (1) est réalisée sous la forme d'un disque plat, transversal à la direction longitudinale de la bande synthétique (1) et raccordé de façon fixe sur celle-ci.

3. Bande suivant la revendication 1 ou la revendication 2, caractérisée en ce que l'anneau de tension (5) est muni, sur sa surface intérieure, de nez d'arrêt (7), dont au moins l'un dépasse dans le canal de bande (6), en empêchant la bande synthétique (1), introduite à l'intérieur, de se déplacer dans le sens opposé à la direction de la tension.

4. Bande suivant l'une quelconque des revendications 1 à 3, caractérisée en ce que l'anneau de tension (5) présente au moins une fente (9) dirigée essentiellement son sens longitudinal.

5. Bande suivant la revendication 4, caractérisée en ce que l'anneau de tension (5) est divisé en segments d'anneaux (8) sur une partie de son extension longitudinale, par les fentes (9) partant de son extrémité en surépaisseur.

6. Bande synthétique supportant une contrainte permanente, avec dispositif autoblocant, pour utilisation en chirurgie et en orthopédie, comportant un anneau de tension (5, 13), traversé, dans le sens longitudinal, par un canal de bande (6), une bande synthétique (1) à élasticité permanente, guidée dans le canal de bande (6) et remplissant essentiellement ce dernier, et des dispositifs de tension (7, 8) empêchant, par un rétrécissement du canal de bande (6), la bande synthétique (1) de se déplacer dans le sens opposé à la direction de la tension, même dans le cas d'un effort de traction, étant entendu que la bande synthétique (1) et l'anneau de tension (5, 13) sont constitués de matière biocompatible, résorbable ou compatible de façon permanente, et étant entendu que la bande synthétique (1) est raccordée à une extrémité de façon fixe avec l'extrémité (14), arrière dans la direction de la tension, de l'anneau de tension (13), caractérisée en ce que la bande synthétique (1) présente des surfaces externes lisses et que l'anneau de tension (13) se rétrécit en formant un cône dans le sens opposé à la direction de la tension de la bande synthétique (1), ou est réalisé cylindrique.

7. Bande suivant la revendication 6, caractérisée en ce que l'anneau de tension (13) est muni, sur sa surface intérieure, de nez d'arrêt (7), dont au moins l'un dépasse dans le canal de bande (6), en empêchant la bande synthétique (1), introduite à l'intérieur, de se déplacer dans le sens opposé à la direction de la tension, et présente, le cas échéant, une surface extérieure aplatie ou cintrée.
